# EUROPEAN PATENT SPECIFICATION

(11) **EP 3 172 973 B1**
(45) Date of publication and mention of the grant of the patent: **08.04.2020**
(21) Application number: 15472004.9
(22) Date of filing: 27.11.2015
(51) Int. Cl.: A23L 33/135, A23C 9/123, A61K 35/744, C12N 1/20, A61P 1/16, A61P 15/02, A61P 37/00

(54) **PROBIOTIC FORMULA, PROCESS OF ITS PREPARATION AND USE**
PROBIOTISCHE FORMEL, VERFAHREN ZU DEREN HERSTELLUNG UND VERWENDUNG
FORMULE PROBIOTIQUE, PROCÉDÉ POUR SA PRÉPARATION ET SON UTILISATION

(43) Date of publication of application: 31.05.2017
(73) Proprietor: DAFLORN Ltd., 9300 Dobrich (BG)
(72) Inventor: ALEKSANDROV, Nikola G., 9300 Dobrich (BG); PENEVA, Daniela P., 9300 Dobrich (BG)
(74) Representative: Stefanova, Stanislava Hristova

(56) References cited:
- EP-A2- 0 795 604
- WO-A1-2005/056028
- WO-A1-2016/030504
- GB-A- 2 509 475
- US-A1- 2004 248 278
- US-A1- 2013 236 600
- US-A1- 2015 320 675

## Description

### Field of invention

The present invention relates to a probiotic formula, process of its preparation and use as a dietary food or food supplement.

### Background

It is known that products, obtained through lactic acid fermentation are healthy food which majority of people consume from their early childhood.

In order to produce these foods, various strains of lactic acid microorganisms, including strains of *Lactobacillus delbrueckii subsp. bulgaricus, Lactobacillus delbrueckii subsp. lactis, Lactobacillus acidophilus, Streptococcus thermophilus* and others, exhibiting proven probiotic properties, are used independently or in combination.

Based on the knowledge that *Lactobacillus delbrueckii subsp. bulgaricus* can cooperate with *Streptococcus thermophilus* during lactic acid fermentation, currently the traditional lactic acid products or isolates of human or animal origin are used as a main source for new strains of probiotic lactic acid microorganisms.

For example, one of the traditional homemade lactic acid products, called katak, is prepared after boiling a milk from a domestic cow at home (like pasteurization), then cooling down the cooked milk to body temperature and putting it in a calf stomach. The stomach is tied and is placed in a well for about 40 days, and then it is ready for consumption. The product resembles a soft cheese with a salient specific taste and is used as a source for further fermentations.

It is also known that in order to obtain a dry lactic acid product, various methods of drying, including lyophilization, are used. They usually include the steps of sterilization of the raw material, acidity adjustment, fermentation and lyophilization. Glycerin, dimethylsulfoxide, sugar, glycols, which have the ability to penetrate into the cell, as well as some polymeric compounds (polyvinyl, pyrrolidone, polyethylene oxide, etc.), which do not penetrate in it, are used as cryoprotectants. Cryoprotectants reduce the effect of crystallization, prevent sticking and denaturation of the macromolecule, help the preservation of the integrity of the cell membrane. It is also known from the studies of Fakhrildin M. at al., High Institute for Infertility Diagnosis and ART, AL-Nahrain University, Baghdad, IRAQ, 2013 on the effect of bee honey as a substituent in medium for cryoprotection on motility, concentration, morphology and agglutination of human gametes after thawing, that replacement of 10% of the cryoprotective medium by bee honey leads to improving of the quality of sperm. The use of bee honey as a cryoprotectant in lyophilization of probiotic cultures, however, is unknown. US2013/0236600 discloses shelf-stable microorganisms. They are cultured to produce a protective shell. The dietary product can be shelf stable for at least 22 months.

The object of the invention is to provide a probiotic formula, process of its preparation and use as a dietary food or food supplement to restore the functions and the structure of liver, to control the symptoms of food allergies, as well as to restore the normal vaginal lactobacillaceae flora of women.

### Summary of the invention

The invention relates to a probiotic formula involving cooperation of probiotic microorganisms, more precisely a cooperation of strains of probiotic microorganisms, isolated as it is from a traditional dairy product in Rhodope mountains, to a process of preparation of the probiotic formula and its use as a dietary food **for** people with impaired liver function and structure; **for** controlling the symptoms of food allergy and **for** recovering the normal vaginal lactobacillaceae flora of women.

In one aspect of the present invention, the cooperation of probiotic microorganisms includes the following original strains of *Lactobacillus delbrueckii* subsp. *bulgaricus* and *Streptococcus thermophillus,* isolated from a traditional dairy product in the Rhodope Mountains:
Strain *Lactobacillus delbrueckii* subsp. *bulgaricus* DMR1, registered in CCM under Nº 8591/ 17.02.2015;
Strain *Lactobacillus delbrueckii* subsp. *bulgaricus* DMR2, registered in CCM under Nº 8592/ 17.02.2015;
Strain *Streptococcus thermophilus* DMR3, registered in CCM under Nº 8591/ 17.02.2015 and
Strain *Streptococcus thermophilus* DMR4, registered in CCM under Nº 8592/ 17.02.2015

Another aspect of the present invention relates to a process of preparation of probiotic formula involving the probiotic cooperation of microorganism strains, isolated from a traditional dairy product according to the invention.

According to a next aspect, the present invention relates to a probiotic formula which after lyophilization and storage for 24 months at a room temperature up to 24°C, contains live cells from the probiotic cooperation according to the invention, in concentration from 1.1x10⁹ cfu/g to 1.6x10⁹ cfu/g, more precisely concentration of live cells of strain *Streptococcus thermophilus* DMR3, registered in CCM under Nº 8591 and strain *Streptococcus thermophilus* DMR4, registered in CCM under Nº 8592 - from 4.1x10⁸ cfu/g to 1.1x10⁹ cfu/g, and also a concentration of live cells of strain *Lactobacillus delbrueckii* subsp. *bulgaricus* DMR1, registered in CCM under Nº 8591 and strain *Lactobacillus delbrueckii* subsp. *bulgaricus* DMR2, registered in CCM under Nº 8592 - from 5.1x10⁸ cfu/g to 8.4 x 10⁸ cfu/g.

Still another aspect of the present invention relates to colonization of microorganisms, contained in the probiotic formula, prepared according to the process of the invention, in the colon, and namely, that they colonize better in the colon and survive twice better under the conditions of the gastrointestinal tract than a control strain.

Another aspect of the present invention relates to the use of the probiotic formula as a dietary food for recovery of the liver functions in patients with hepatic impairment.

Another aspect of the present invention relates to evidences for the colonization of strain *Lactobacillus delbrueckii* subsp. *bulgaricus* DMR1, registered in CCM under Nº 8591 and strain *Lactobacillus delbrueckii* subsp. *bulgaricus* DMR2, registered in CCM under Nº 8592 and strain Streptococcus thermophilus DMR3, registered in CCM under Nº 8591 and strain *Streptococcus thermophilus* DMR4, registered in CCM under Nº 8592 in vagina of women and the use of the probiotic formula according to the invention for restoration of the normal vaginal microflora. The aspect of the present invention, relating to the use of the probiotic formula to control the symptoms of food allergies is of particular significance. The invention is as defined by the appended claims.

### Brief description of the drawings

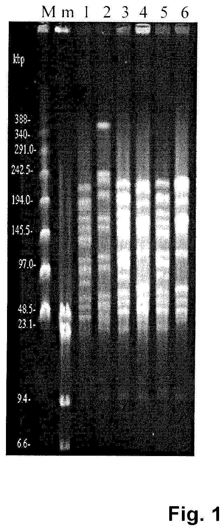

**Fig. 1****:** shows the macrorestriction profile, obtained after restriction with Smal enzyme of genomic DNA of isolates *Streptococcus thermophilus* from sample STM, where: Lane 1-Sample STM, isolate *Streptococcus thermophilus* STMT1; Lane 2 - Sample STM, isolate *Streptococcus thermophilus* STMT2; Lane 3- Sample STM, isolate *Streptococcus thermophilus* STMT3; Lane 4- Sample STM, isolate *Streptococcus thermophilus* STMT4; Lane 5 - Sample STM, isolate *Streptococcus thermophilus* STMT5; Lane 6- Sample STM, isolate *Streptococcus thermophilus* STMT6; m - pulsed-field marker 0,1-200 kbp (Sigma); M- pulsed-field marker 50-1000 kbp (Sigma).

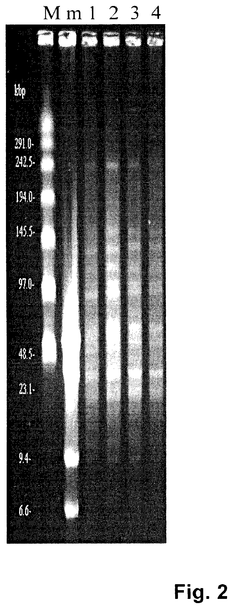

**Fig. 2****:** shows the macrorestriction profile, obtained after restriction with *Xho*I enzyme of genomic DNA of isolates *Lactobacillus bulgaricus* from sample STM, where: Lane 1- Sample STM, isolate *Lactobacillus bulgaricus* STML 1; Lane 2- Sample STM, isolate *Lactobacillus bulgaricus* STML 2; Lane 3- Sample STM, isolate *Lactobacillus bulgaricus* STMM 3; Lane 4-Sample STM, isolate *Lactobacillus bulgaricus* STMM 4; m- pulsed-field marker 0,1-200 kbp (Sigma); M- pulsed-field marker 50-1000 kbp (Sigma).

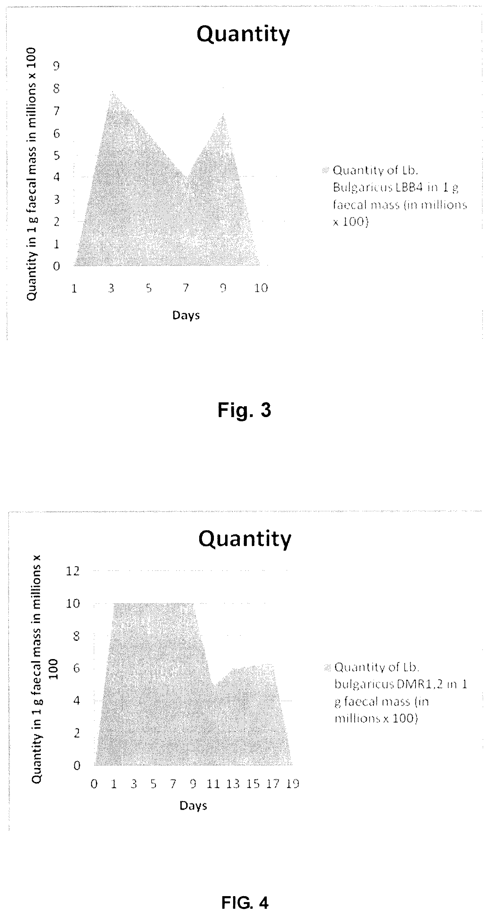

**Fig. 3** presents a diagram of the content of strain *Lactobacillus bulgaricus LBB4* in the faeces of children aged from 1 to 3 years who have taken daily 100 g yoghurt, standardized to BDS, for 3 days.
Fig. **4** presents a diagram of the content of strain *Lactobacillus bulgaricus DMR1* and strain *Lactobacillus bulgaricus DMR2* in the faeces of children aged from 1 to 3 years who have taken daily 3 g probiotic formula, obtained according to the invention, for 3 days.

### DETAILED DESCRIPTION OF THE INVENTION

Specimens of the microorganism strains, contained in the probiotic cooperation of microorganisms, isolated as it is from a traditional dairy product in the Rhodope mountains, according to the invention, were deposited on 17.02.2015 in the Czech Collection of Microorganisms (CCM) under the terms and conditions of the Budapest Treaty on the International Recognition of the Deposit of Microorganisms for the Purposes of Patent Procedure. Each of them has a registration number, respectively, as provided below:
Strain *Lactobacillus delbrueckii* subsp. *bulgaricus* DMR1, registered in CCM under Nº 8591
Strain *Lactobacillus delbrueckii* subsp. *bulgaricus* DMR2, registered in CCM under Nº 8592
Strain *Streptococcus thermophilus* DMR3, registered in CCM under Nº 8591 and
Strain *Streptococcus thermophilus* DMR4, registered in CCM under Nº 8592.

As shown in Example 1 below, the species affiliation of the rods has been established through the ARDRA method (amplified ribosomal DNA restriction analysis) following *Giraffa et al.* The species affiliation of the cocci forms has been established by species-/genus-specific PCR following *Lick et al., Nomura et al.* and *Ke et al.* Strains *Lactobacillus delbrueckii* subsp. *bulgaricus* ATCC 11842, *Lactobacillus helveticus* DSM20075, *L. delbrueckii* subsp. *lactis* DSM 20072, *Streptococcus thermophilus* DSM 20617; *Lactococcus lactis* subsp. *lactis* DSM 20481 and *Enterococcus faecalis* LMG 2937 have been used as reference cultures.

The results about the number of the grown colonies in environments M17 agar and MRS agar show that all the cocci isolates belong to the *Streptococcus thermophilus* species and reach quantity up to 4,9 x 10⁸ cfu/g, while all the isolates of the rods belong to the *Lactobacillus delbrueckii* subsp. *bulgaricus* and reach a quantity up to 8,4 x 10⁸ cfu/g. Under the same analytical conditions (in environments MRS and M17) enterococci, bacilli or yeasts have not been detected.

Distinguishing between the various microorganism strains in the samples of the probiotic cooperation, as shown in Example 1 of the present invention, was made by macrorestriction analysis using Pulsed Electrophoresis of the CHEF-DR II BioRad system. For the restriction analysis of the genomic DNA the enzymes *Sma*I and *Xho*I were used for the cocci and the rods respectively.

The study regarding the strain composition of the samples after macrorestriction analysis with Smal enzyme of *Streptococcus thermophilus* showed the presence of strain *Streptococcus thermophilus* DMR3 and strain *Streptococcus thermophilus* DMR4 (Fig.1), and the macrorestriction analysis with *Xho*I enzyme of *Lactobacillus delbrueckii* subsp. *bulgaricus* showed the presence of strain *Lactobacillus delbrueckii* subsp. *bulgaricus* DMR1 and strain *Lactobacillus delbrueckii* subsp. *bulgaricus* DMR2 (Fig. 2).

In order to obtain the probiotic formula including the probiotic cooperation of microorganisms, according to the invention, the strains *Lactobacillus delbrueckii* subsp. *bulgaricus* DMR1, registered in CCM under Nº 8591, *Lactobacillus delbrueckii* subsp. *bulgaricus* DMR2, registered in CCM under Nº 8592, *Streptococcus thermophilus* DMR3, registered in CCM under Nº 8591 and *Streptococcus thermophilus* DMR4, registered in CCM under Nº 8592 are first captured from a traditional home made dairy product, specific for the Rhodopes area and are multiplied in order to produce biomass. For that purpose, standardized and pasteurized cow's milk with fat content 3.6% or sterilized pasteurized goat's milk with fat content 3.2% is fermented by a traditional dairy product, isolated from the Rhodopes area, in ratio 1:0.005 and is placed in a water-bath at a temperature of 43° - 45°C for 24 hours and after that a white biomass with soft consistency is released on the surface.

The obtained biomass is used then to prepare the probiotic formula. For this purpose a liquid starter culture is prepared first, as first of all a 17% water solution of dry standardized full fat or skimmed cow's milk is prepared. In some cases, fresh goat's milk with fat content 3.2% is used. This solution of cow's milk or fresh goat's milk is subjected to sterilization at 95°C - 96°C for 30 minutes and after that is cooled down to 45°C. The pH value is observed from 6.5 to 6.7. Adjustment of pH is not required at that value. Fermentation with the biomass, obtained as described above, comes next at the ratio of the rehydrated cow's milk or fresh goat's milk: starter culture 1:0.001 and then fermentation for 18 hours at 43°C - 45°C in order to obtain a liquid starter culture. This liquid starter culture is added to a water solution of cow's milk or goat's milk, obtained as described above, at a ratio milk: liquid starter culture 3:1 and cryoprotectant, which in that case is previously heated bee honey at a ratio 50:1, is added to this solution. The obtained liquid product is homogenized at a temperature of 41°C - 44°C and is left to ferment for 3 hours, after which is cooled down to 24°C - 25°C and is subjected to lyophilization for 72 hours including freezing at a temperature of -41°C and sublimation until a temperature of 32°C is reached. The obtained finished dry product is the probiotic formula according to the invention. The latter is stored at a room temperature up to 24°C and is used as a dietary food or a food supplement for dietetic nutrition, medical product for a topical vaginal application, and also as a starter culture for subsequent fermentations of milk after preliminary rehydration in ratio 1:0.5 until a liquid starter culture is obtained.

In particular, in order to use the obtained probiotic formula in a dry form as a starter culture for next fermentations, it is rehydrated in ratio water: dry product 1:0.5 until a liquid starter culture is obtained. This liquid starter culture is added to a water solution of cow's milk or goat milk, obtained as described above in ratio milk: liquid starter culture 3:1 and then a cryoprotectant, which in that case is a previously heat treated bee honey, is added to this solution in ratio 50:1. The obtained liquid product is homogenized at a temperature of 41°C - 44°C and is left to ferment for 24 hours and then is cooled down to 24°C - 25°C and is subjected to lyophilization for 72 hours. The latter includes freezing at a temperature of -41°C and sublimation until a temperature of 32° C is reached. The obtained products are stored at a room temperature.

The studies of the survival of the microorganisms after lyophilization of the product, carried out in "DNA analyses" LAB of ELBI Bulgaricum show that the cooperation of probiotic strains of lactic acid microorganisms, contained in the probiotic formula according to the invention, preserves its viability during a prolonged storage at a room temperature. The samples stored for 24 months at a room temperature, contain live microorganisms in quantity close to that at the date of manufacture. The latter is of particular importance with regard of the supply of the dietary products, produced according to the method of the invention, on the market. The products, obtained according to the invention, are conditionally designated P1 and P2.

Colonization of the strain *Lactobacillus delbrueckii* subsp. *bulgaricus* DMR1 *and* strain *Lactobacillus delbrueckii* subsp. *bulgaricus* DMR2 in the colon is studied in the Medical University of Sofia. The double blind randomized clinical study, carried out on two groups of 5 children aged from 1 to 3 years, show that the strain *Lactobacillus delbrueckii* subsp. *bulgaricus DMR1* and strain *Lactobacillus delbrueckii subsp. bulgaricus DMR2* in the probiotic formula, obtained through the method as per the invention, are colonized better in the colon and survive twice better under the conditions of the gastrointestinal tract as compared to the control strain *Lactobacillus delbrueckii subsp. bulgaricus LBB4* in yoghurt.

The probiotic formula, prepared by the process according to the invention, is a dry dietary lactic acid product applicable as a dietetic food or a food supplement independently or in combination with plant extracts or essential oils for restoration of the liver functions, to control the symptoms of food allergy and as a medical device for recovery of the normal vaginal microflora of women In order to restore the functions of the liver in case of injuries due to alcohol, the patients have taken 15 g twice a day before meal the probiotic formula, prepared by the process according to the invention from dry whole cow's milk and 3 to 6 capsules, containing the product, obtained according to the invention in combination with a dry extract of milk thistle (*Silybum marianum*)*,* standardized to silymarin, dextrose monohydrate and magnesium stearate. The ratio in 1 cellulose capsule, intended for oral administration, is 15:10:1:1. The clinical observation shows that adding a nutritional therapy with the probiotic formula (including strains of *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4), prepared by the process according to the invention, to the complex treatment regimen and the combination of the probiotic formula, prepared according to the invention, with dry extract of milk thistle leads to restoration of the functions of the liver cell in patients with alcoholic disease. In that case the products are conditionally designated as P5 and P4 (capsule form). In cases of chronic alcohol intoxication the liver injuries are leading and therefore it is used as a model of chronic liver injuries.

Furthermore - a daily intake of 60 g of the probiotic formula according to the invention, for 6 months has led to a full recovery of the functions and the structure of the liver of a patient with liver cirrhosis - a disease which in medicine is considered to be irreversible and leads to death. A patient, aged 28 years with liver cirrhosis that has been established by biochemical and ultrasound examinations, through computer tomography and histologically (liver biopsy) in two leading clinics in Bulgaria and Germany, needs no liver transplantation due to including a nutritional therapy with product P5, obtained as per the invention, in addition to the standard treatment regimen. Only for six months the patient has almost completely recovered clinically, also her blood and instrumental tests have been improved and her working ability has restored. Incidence of allergic diseases is increasing in all age groups. One of the most spread allergies is food allergy. For example, for the last decade, frequency of allergy to peanuts has doubled. Food allergy is defined as an immune mediated hypersensitivity reaction to food proteins which are harmless but are alien to the human immune system. Food allergy is manifested by various symptoms such as lacrimation, runny nose, rashes, itchy skin, gastroenterocolitis, headache. Studies of the inventors in the last years have found out other symptoms, occurring as a result of food allergy, which have not been associated with allergic diseases so far - hair loss, dandruff, increased blood pressure, periodontal disease, changes in hormonal status of patients (increased level of prolactin in women) and others.

After establishing the type of the food allergy, stopping of the consumption of foods containing these food allergens and intake of the probiotic formula according to the invention in combination with Bulgarian rose oil, dextrose monohydrate and magnesium stearate was recommended. The administration of the probiotic formula, according to the invention, in a dose of 2 to 6 capsules daily for a period of 2 months, leads to abatement of the clinical symptoms of food allergy for a period of 2 to 6 weeks depending on the severity of allergy. Stopping of the hair loss and dandruff, normalization of the blood pressure without changing the antihypertensive therapy, reduction of bleeding and inflammation of gums and normalization of prolactin levels was observed in patients. Emergence of new hair was also observed in patients with hair loss. In that case, the content of the dietary dairy product, according to the invention, in combination with Bulgarian rose oil, dextrose monohydrate and magnesium stearate in one capsule is in ratio of 25: 1:2: 3 (250 mg dry lactic acid product from goat's milk, 10 mg Bulgarian rose oil, 20 mg dextrose monohydrate and 30 mg magnesium stearate), as this product in that form is conditionally designated as P6.

The probiotic formula as per the invention, is applicable for restoration of the normal vaginal microflora of women with diagnosed vaginal discharge in combination with dextrose monohydrate in ratio 25:7 (250 mg dry product from skimmed cow's milk and 70 mg dextrose monohydrate), in rapidly soluble vegetable capsules for topical application during and 5 days after the etiological treatment of vaginal discharge. In this case the product is conditionally designated as P3.

The beneficial effect of the probiotic formula as per the invention on the vaginal flora of women with vaginal discharge has been proven by a clinical study, carried out in MC Evrozdrave and MC Gynart MMA, Sofia for a period of 23 months. The clinical study involved 32 women aged between 18 and 46 years with a regular menstrual cycle that were not pregnant. The results detailed in Example 9 below, unambiguously show the effect of the product, containing strains *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4 on the restoration of the normal lactobacillus flora of women, treated for vaginal discharge.

The invention is illustrated by the following examples, without limiting its scope.

### Example 1: Strain capturing

*Lactobacillus delbrueckii subsp. bulgaricus DMR1,* registered in CCM under Nº 8591, *Lactobacillus delbrueckii subsp. bulgaricus DMR2,* registered in CCM under Nº 8592, *Streptococcus thermophilus DMR3,* registered in CCM under Nº 8591 and *Streptococcus thermophilus DMR4,* registered in CCM under Nº 8592 are captured from a traditional lactic acid product, specific for the Rhodopes area.

Generations inherit a method for production of a dairy product, as fresh cow's milk is boiled at home, resembling pasteurization, after which the boiled milk is cooled down to a body temperature, it is placed in a calf stomach and 5.0 g of the lactic acid product, prepared by the same method 40 days before that, are added to it. The stomach is tied and is placed in a well. It matures/ferments for 40 days and then is ready for consumption. The product is like the traditional product named katak. In order to capture the strains, 5.0 g of this traditional lactic acid product, specific for the Rhodopes area, are added to 1.0 l standardized and pasteurized cow's milk with 3.6% fat and are heated in a water bath at 43-45° C for 24 hours. At the end of that period a white biomass is released on the surface of the mixture.

### Example 2: Proving the species and strain composition of the cooperation of probiotic microorganisms

Establishing the species composition of the probiotic cooperation of the invention has been carried in the "DNA Analysis" Lab of ELBI Bulgaricum EAD. The species affiliation of the rods has been established through the ARDRA method (amplified ribosomal DNA restriction analysis) following *Giraffa et al.;* the species affiliation of the cocci has been established by the species-/genus-specific PCR following *Lick et. al., Nomura et al. and Ke et al.; Lactobacillus delbrueckii* subsp. *bulgaricus* ATCC 11842, *Lactobacillus helveticus* DSM20075, *L. delbrueckii* subsp. *lactis* DSM 20072, *Streptococcus thermophilus* DSM 20617; *Lactococcus lactis* subsp. *lactis* DSM 20481 and *Enterococcus faecalis* LMG 2937 have been used as reference cultures.

The results from the studies show that the species composition of the samples is limited to *Lactobacillus delbrueckii subsp. bulgaricus* and *Streptococcus thermophilus,* as under the same analytical conditions (on environments MRS and M17) enterococci, bacilli or yeasts have not been established.

Distinguishing the separate strains between the isolates of the samples was made by macrorestriction analysis using the Pulsed Electrophoresis of the CHEF-DR II BioRad system. For the restriction analysis of the genomic DNA the enzymes Smal and *Xho*I were used for the cocci and the rods respectively. The study with regard to the strain composition of the samples after the macrorestriction analysis with the Smal enzyme of *Streptococcus thermophilus* showed the presence of the strain *Streptococcus thermophilus DMR3* and strain *Streptococcus thermophilus DMR4* (Fig. 1), and the macrorestriction analysis with the *Xho*I enzyme of *Lactobacillus delbrueckii* subsp. *bulgaricus* - the presence of the strain *Lactobacillus delbrueckii subsp. bulgaricus DMR1* and strain *Lactobacillus delbrueckii subsp. bulgaricus DMR2* (Fig. 2). Tests have been carried out for a sample formally designated as STM. It is a soft-texture biomass, obtained by capturing the microorganisms of a traditional dairy product in the Rhodopes in July 2013. The sample STM has been tested for species and strain composition and quantitative values of cocci and rods in August 2013. The results are presented in Table 1 below:

**Table 1**

| Sample | *Lactobacillus delbrueckii* ssp. *bulgaricus* | *Streptococcus thermophilus* |
|---|---|---|
| STM | 8,4 x 10⁸ | 4,9 x 10⁸ |

### Example 3: Preparation of a probiotic formula, containing Lactobacillus delbrueckii subsp. bulgaricus DMR1, Lactobacillus delbrueckii subsp. bulgaricus DMR2, Streptococcus thermophilus DMR3 and Streptococcus thermophilus DMR4

In order to prepare the probiotic formula with the probiotic cooperation, containing *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4, described in details in Example 1 above, first the microorganisms, isolated from a traditional lactic acid product, specific for the Rhodopes area, are captured and multiplied in order to obtain biomass. For that purpose, 1.0 l standardized and pasteurized cow's milk with 3.6% fat or sterilized and pasteurized goat's milk with 3,2% fat is fermented by 5 g of a traditional lactic acid product, specific for the Rhodopes area. The container with milk and the specific product are placed in a water bath at a temperature of 43° - 45° C for 24 hours, at the end of that period a white biomass with soft consistency is released on the surface of the mixture.

The obtained biomass is used to obtain a liquid starter culture. 5.0 l 17% water solution of dry standardized full-fat or skimmed cow's milk or fresh goat's milk with 3.2% fat is prepared. This solution or fresh milk is subjected to sterilization at a temperature of 95°C - 96°C for 30 minutes, and then it is cooled down to 45°C. The value of pH from 6.5 to 6.7 is observed. Adjustment of pH is not required at that value. 5.0 g from the biomass, obtained as described above, is added to 5.0 l from this solution of cow's milk or fresh goat's milk and is left to ferment for 18 hours at 43°C - 45°C to obtain a liquid starter culture. Then the cryoprotectant bee honey is prepared. Bee honey is heated in a water bath until boiling and is left to cool down to 24°C-25°C. The obtained liquid starter culture is added to a previously prepared and sterilized at 95°C-96°C for 30 minutes 17% water solution of dry standardized full-fat or skimmed cow's milk or fresh goat's milk with 3.2% fat in a ratio milk:liquid starter culture 3:1. The cryoprotectant (cooled bee honey) is added to this solution in ratio solution:honey 50:1. The obtained liquid product is homogenized at a temperature of 41°C - 44°C and is left to ferment for 3 hours and then is cooled down to 24°C - 25°C and is subjected to lyophilization for 72 hours. The latter includes freezing at a temperature of -41°C and sublimation until temperature of 32°C is reached. The thus obtained dry product which is the probiotic formula according to the invention, is stored at a room temperature and is used as food or a food supplement for a dietetic nutrition, a medical preparation for a topical vaginal application, and also as a starter culture for fermentation in next preparations of the product, according to the method of the invention.

For the next fermentation, the obtained finished dry product is rehydrated in ratio water:dry product 1:0.5 until a liquid starter culture is obtained. This liquid starter culture is added to a water solution of cow's or goat's milk, obtained as described above, in ratio milk: liquid starter culture 3:1 and a cryoprotectant, which in that case is previously thermally processed bee honey, is added to this solution in ratio 50:1. The thus obtained liquid product is homogenized at a temperature of 41°C - 44°C and is left to ferment for 24 hours, after which is cooled down to 24°C - 25°C and is subjected to lyophilization for 72 hours. The latter includes freezing at a temperature of -41°C and sublimation until a temperature of 32° C is reached. The obtained products are stored at a room temperature.

### Example 4: Survival in time of the strains in the probiotic formula, obtained according to the invention when stored at a room temperature 24°C.

The survival of the microorganisms after lyophilization of the product has been established in DNA Analysis Lab of ELBI Bulgaricum, Sofia, Bulgaria, by testing the obtained dry product for CFU per gram immediately after obtaining and after storage at a temperature of 24° C for 24 months. The tests have been carried out for two samples, conditionally designated as P1 and P2, where the sample P1 is a dry dairy product, obtained through the method of the invention in July 2013, and sample P2 is a dry dairy product, obtained through the method of the invention in August 2013. Samples P1 and P2 were studied for species and strain composition and quantitative values of cocci and rods respectively in August 2013, conditionally designated as P11 and P21 and in August 2015, conditionally designated as P12 and P22. The two samples P1 and P2 are stored at a room temperature 20 - 24°C after manufacture.

The results are presented in Table 2 below.

**Table 2**

| Sample | *Streptococcus thermophilus* | *Lactobacillus delbrueckii* ssp. *bulgaricus* | Total number of lactic acid microorganisms |
|---|---|---|---|
| P11 | 4,9 x 10⁸ | 8,4 x 10⁸ | 1,3 x 10⁹ |
| P21 | 1,1 x 10⁹ | 6,2 x 10⁸ | 1,6 x 10⁹ |
| P12 | 4,1 x 10⁸ | 7,2 x 10⁸ | 1,1 x 10⁹ |
| P22 | 1 x 10⁹ | 5,1 x 10⁸ | 1,5 x 10⁹ |

The results from the analyses, carried out in DNA Analysis Lab of ELBI Bulgaricum, show that the cooperation of probiotic strains of lactic acid microorganisms, contained in samples P1 and P2, stored for 24 months (from August 2013 to August 2015) at a temperature of 20°C- 24°C (respectively P12 and P22), contains live microorganisms in amounts close to these at the date of production (respectively P11 and P21). Thus the probiotic cooperation of strains of lactic acid microorganisms in the product, obtained according to the invention, retains its viability during a prolonged storage at a room temperature. The latter is of paramount importance for the products, produced by using the technology of the invention, offered on the market.

### Example 5: Colonization in the colon.

In order to study the colonization of strains *Lactobacillus delbrueckii* subsp. *bulgaricus* DMR1 and *Lactobacillus delbrueckii* subsp. *bulgaricus* DMR2 in the Medical University of Sofia, a double blind randomized clinical observation was carried out on 2 groups of 5 children aged from 1 to 3 years. The first group has taken daily 100 g yoghurt, standardized to BDS, containing 1x10⁹ CFU of strain *Lactobacillus delbrueckii* subsp. *bulgaricus LBB4* for 3 days. The second group has taken daily 3 g rehydrated product, obtained from dry full-fat cow's milk by the method of the invention, containing 1x10⁹ CFU of strains *Lactobacillus delbrueckii* subsp. *bulgaricus* DMR1 and *Lactobacillus delbrueckii* subsp. *bulgaricus* DMR2 for 3 days. The presence of strain *Lactobacillus delbrueckii subsp. bulgaricus LBB4* has been established up to the 10^{th} day after the consumption of yoghurt, maximum to 7,8 x 10⁸ CFU/g faecal mass in the first group. It is presented on Figure 3.

The presence of strains *Lactobacillus delbrueckii* subsp. *bulgaricus* DMR1 and *Lactobacillus delbrueckii* subsp. *bulgaricus* DMR2 has been established in the second group to the 19^{th} day after consumption of the probiotic formula, maximum to 10 x 10⁸ CFU/g faecal mass, as that content persists for 7-8 days. It is shown on Figure 4.

The results show that the strains *Lactobacillus delbrueckii* subsp. *bulgaricus* DMR1 and *Lactobacillus delbrueckii* subsp. *bulgaricus* DMR2 in the lactic acid product, obtained by the method of the invention, colonize much better and survive twice longer than the other strain *Lactobacillus delbrueckii* subsp. *bulgaricus* LBB4 in yoghurt.

**Example 6:** Effect of a probiotic formula, containing cooperation of strains of *Lactobacillus delbrueckii subsp. bulgaricus DMR1, Lactobacillus delbrueckii subsp. bulgaricus DMR2, Streptococcus thermophilus DMR3* and *Streptococcus thermophilus DMR4,* prepared by the method according to the invention from dry full-fat cow's milk in combination with a product in capsules, obtained according to the invention and dry extract of milk thistle, on chronic liver injury in patients with alcoholic disease.

In cases of chronic alcohol intoxication liver damage is leading, so it is used as a model of a chronic liver injury.

For the purpose of the study the patients have taken twice a day before meal 15 g product, obtained through the method of the invention from dry full-fat cow's milk, formally called P5. At the same time the patients have taken 6 cellulose capsules for oral use, as each capsule /270 mg/ contains 150 mg product, obtained according to the invention from full-fat cow's milk, 100 mg dry extract of milk thistle, standardized to silymarin, 10 mg dextrose monohydrate and 10 mg magnesium stearate. This product is conditionally designated P4.

The clinical observation has been carried out in MC Evrozdrave- Bulgaria, Sofia for a period of 20 months. Object of the study were 32 patients with chronic alcohol abuse. The patients were aged between 22 and 66 years. 22 of them were men (68,8%) and 10 were women (31.2%). In 9 (28.1%) of the patients the duration of the alcohol abuse is up to 5 years, in 18 (56.3%) - it is up to 10 years and in 5 (15.6%) - over 10 years. Alcohol used by the addicted patients is mostly concentrated /vodka, whiskey, brandy/ and is in amount of 300 ml to 900 ml per 24 hours. The diagnosis alcohol disease of all patients has been established on the basis of medical history, study of the alcohol amount in serum, changes in biochemical indicators - increased activity of gamma-glutamine transpeptidase (GGTP), aspartate aminotransferase (ASAT), alanine aminotransferase (ALAT) and ultrasonic diagnosis of liver (USD). Complications of the liver, resulting from long-term use of alcohol, are clinically manifested by heaviness in the area of epigastrium and the right sub costal zone, nausea, flatulence, jaundice. The liver is painful when palpated and is with dense to solid consistency and rounded edge. Table 3 and Table 4, respectively, present ultrasound image of liver upon hospitalization of the patients for treatment and values of the liver enzymes (GGTP, ASAT, ALAT), and Tables 5 and 6 show the change of the ultrasound image of liver respectively for a treatment period of 6 months and the change in the values of the liver enzymes for a treatment period of 6 months.

**Table 3**

| Ultrasound image of liver upon hospitalization of the patients for treatment | |
|---|---|
| **Ultrasonographic evidence of liver** | **Number of patients in %** |
| No abnormalities in ultrasound data | 28,1% (9 patients) |
| Ultrasound evidence of hepatic steatosis | 53,1% (17 patients) |
| Ultrasound evidence of chronic hepatitis | 18,8% (6 patients) |

**Table 4**

| Values of the liver enzymes (GGTP, ASAT, ALAT) upon hospitalization of the patients for treatment | |
|---|---|
| **Values of the liver enzymes** | **Number of patients in %** |
| Values in the range of referents | 12,5% (4 patients) |
| Values increased to 100% | 31,3% (10 patients) |
| Values increased from 100% to 300% | 53,1% (17 patients) |
| Values increased over 300% | 3,1% (1 patient) |

The patients were randomly divided into 2 groups. The first group included 15 patients, who have taken medications of a complex treatment regimen, involving infusion of monosaccharide and aqueous-salt solutions, nootropic and psycho energetic remedies (Piracetam), neuroprotective agents (Nivalin, Milgamma, Vitamin B6), hepatoprotective agents (Transmetil, Carsil, Legalon), phenothiazine preparations (Carbamazepin), psychotherapeutic rehabilitation and symptomatic treatment if necessary. The second group included 17 patients, who received the complex treatment regimen, described above, and have taken product P5 at a dose of 15 g twice daily orally before meal for a period of 6 months and a product in capsules P4 at a daily dose of 2 capsules three times for a period of 3 months and the next 3 months - at a daily dose of 1 capsule three times a day 15-30 minutes before meal. The results are summarized in Tables 5 and 6 below.

**Table 5**

| Change in the ultrasound image of liver for a treatment period of 6 months | | | | | | |
|---|---|---|---|---|---|---|
| | **First group (15 patients who have not taken P5 and P4)** | | | **Second group (17 patients who have taken P5 and P4)** | | |
| **Liver ultrasound data** | **Hepatic steatosis** | **Chronic hepatitis** | **No abnormalities in the ultrasound image** | **Hepatic steatosis** | **Chronic hepatitis** | **No abnormalities in the ultrasound image** |
| | **Number of patients in %** | **Number of patients in %** | **Number of patients in %** | **Number of patients in %** | **Number of patients in %** | **Number of patients in %** |
| Upon reception | 8 (53,3%) | 3(20%) | 4 (26,7%) | 9 (52,9%) | 3 (17,6%) | 5 (29,4%) |
| After 3 months | 7 (46,7%) | 3 (20%) | 5 (33,3%) | 6 (35,3%) | 2 (11,8%) | 9 (52,9%) |
| After 6 months | 6 (40%) | 2 (13,3%) | 7 (46,7%) | 3 (17,6%) | 1 (5,9%) | 13 (76,5%) |

**Table 6**

| Change in the values of the liver enzymes for a treatment period of 6 months | | | | | | | | |
|---|---|---|---|---|---|---|---|---|
| | **First group (15 patients who have not taken P5 and P4)** | | | | **Second group (17 patients who have taken P5 and P4)** | | | |
| **Values of liver enzymes (GGTP, ASAT, ALAT)** | **In the range of referents** | **Increased to 100%** | **Increased from 100% to 300%** | **Increased over 300%** | **In the range of referents** | **Increased to 100%** | **Increased from 100% to 300%** | **Increased over 300%** |
| | **Number of patients in %** | **Number of patients in %** | **Number of patients in %** | **Number of patients in %** | **Number of patients in %** | **Number of patients in %** | **Number of patients in %** | **Number of patients in %** |
| **Upon admission** | 2 (13,4%) | 5 (33,3%) | 8 (53,3%) | 0 | 2 (11,8%) | 5 (29,4%) | 9 (52.9%) | 1 (5,9%) |
| **After 3 months** | 3 (20%) | 7 (46,7%) | 5 (33,3%) | 0 | 4 (23,5%) | 10 (58,8%) | 3 (17,7%) | 0 |
| **After 6 months** | 5 (33,3%) | 8 (53,3%) | 2 (13,4%) | 0 | 11 (64,7%) | 5 (29,4%) | 1 (5,9%) | 0 |

The clinical observation shows that adding a nutritional therapy with the probiotic formula, containing strains of *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4, prepared by the method of the invention from dry full-fat cow's milk and a product that is a combination of a product, obtained by the method of the invention from dry full-fat cow's milk and silymarin, to the complex treatment regimen leads to a more rapid recovery of the functions of the liver cells in patients with alcoholic disease.

**Example 7:** Effect of a product, containing a cooperation of strains of *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4, obtained through the method according to the invention from dry fuul-fat cow's milk, observed in a case of liver cirrhosis of probable ethylic genesis, accompanied by icterus, ascites and anasarca.

The clinical observation has been carried out by prof. Dr. Nikola Alexandrov, MD, PhD and Dr. Daniela Petrova PhD on a woman of 28 years, diagnosed and treated consecutively in Clinic of Gastroenterology in Germany, twice in MMA - Sofia Hospital, Clinic of intensive therapy and MC Evrozdrave.

The patient was admitted to hospital in Germany on 05.03.2012. The conducted biochemical blood tests show - bilirubin - 10,8 mg/dl /norm below 1,1 mg/dl/, ASAT - 273U/I /norm below 35 U/I/, GGTP - 777 U/I /at a rate below 38 U/I/, albumin - 1,78 g/dl /at a norm of 3,5 - 5 g/dl/; data from computer tomography and liver biopsy for a clear inflammatory activity in the parts and portal fields of liver, pericellular portal fibrosis forming septa, opening cirrhosis transformation of liver. The patient received treatment with Pantozol and Folsan. The patient was discharged from the Clinic of Gastroenterology in Germany on 24.03.2012 and was directed to Bulgaria for a liver transplantation. Data is from medical history.

She was admitted to MMA Hospital Sofia, Bulgaria in Clinic of intensive therapy on 02.04.2012 with symptoms of decompensated liver cirrhosis. The biochemical tests show total bilirubin - 169 µmol/l /rate up to 21 µmol/l/, direct bilirubin - 130 /rate up to 4-5 µmol/l/, ASAT - 279 U/I /rate up to 41 U/I/, albumin - 24 g/l /norm 33-45 g/l/; data from computer tomography and liver biopsy for enlarged liver with decreased density of parenchyma - cirrhotic converted liver. Treatment with Transmetil, Hepa-Merz, Human Albumin and symptomatic agents has been carried out. In the Clinic of intensive therapy started a nutritional therapy with 60 g daily of a product, obtained by the method of the invention from dry full-fat cow's milk, conditionally designated P5. The patient was discharged from hospital with a good progress on 18.04.2012 - the biochemical tests total bilirubin - 70 µmol/l /norm up to 21 µmol/l/, direct bilirubin - 26 /norm up to 4-5 µmol/l/, ASAT - 135 U/I /norm up to 41 U/I/, albumin - 30 g/l /norm 33-45 g/l/.

The patient continued her treatment at home with Transmetil, vitamins, symptomatic agents and a product, obtained by the method according to the invention from dry full-fat cow's milk, conditionally called P5 at a dose of 60 g daily.

The woman was admitted for a second examination on 17.09.2012 to MMA Hospital Sofia, Clinic of intensive therapy. The biochemical tests are total bilirubin - 16 µmol/l /norm up to 21 µmol/l/, direct bilirubin - 5 /norm up to 4-5 µmol/l/, ASAT - 29 U/I /norm up to 41 U/I/, albumin - 45 g/l /norm 33-45 g/l/; data from computer tomography and ultrasound diagnosis of hepatic steatosis, a moderate liver increase, diffusively increased echogenicity without focal changes and liver cirrhosis. Data are from epicrisis.

The clinical study shows that adding a nutritional therapy with a probiotic formula, containing *Lactobacillus delbrueckii subsp. bulgaricus DMR1, Lactobacillus delbrueckii subsp. bulgaricus DMR2, Streptococcus thermophilus DMR3* and *Streptococcus thermophilus DMR4,* obtained by the method of the invention from dry full-fat cow's milk to the complex treatment regimen leads to a reverse development of liver cirrhosis, rapid recovery of the functions of the liver cells and successful control of hypoproteinaemia. The patient with liver cirrhosis, proven biochemically, by ultrasound, by computer tomography and histologically /liver biopsy/ in two leading Hospitals in Bulgaria and Germany, does not need liver transplantation due to the included nutritional therapy with product P5 into the standard treatment regimen. Only for six months the patient has recovered almost completely clinically as well as in regard of blood and instrumental tests and her working ability has been restored. In 2015 she gave birth to a child, born alive.

**Example** 8: Effect of a combination of a product, containing cooperation of strains of *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4, obtained by the method according to the invention from goat's milk and Bulgarian rose oil, obtained through classic technology from Bulgarian oilseed rose Rosa Damascena, on patients with food allergies.

For the purpose of the study, a product, obtained through the method according to the invention from goat milk, is put together with Bulgarian rose oil and dextrose monohydrate in vegetable capsules for oral use. Content in 1 capsule: 250 mg dry lactic acid product from goat's milk, 10 mg Bulgarian rose oil, 20 mg dextrose monohydrate and 30 mg magnesium stearate, as this product in that form is conditionally designated P6.

Incidence of allergic diseases is increasing in all age groups. One of the most widespread allergies is the food allergy. For example, allergy to peanuts has doubled in the last decade. Food allergy is defined as an immunologically mediated hypersensitivity reaction to food proteins that are harmless, but alien to the human immune system. Food allergy is manifested by various symptoms such as lacrimation, runny nose, rashes, itchy skin, gastroenterocolitis, headache. In the last years we found out other symptoms, occurring as a result of food allergy - hair loss, dandruff, increased blood pressure, periodontal disease, change in the hormonal status of the patients /increased prolactin in women/. These symptoms have not been associated with allergic diseases so far.

The clinical observation has been carried out in MC Evrozdrave - Bulgaria, Sofia and Sofilab Laboratory for a period of 2 years from June 2013 to July 2015. Object of the study were 23 patients aged from 20 to 70 years. All patients were with different clinical manifestations of allergic reactions, described above. Immune tests for food allergens through the ELISA method - immune modulation to determine the number of specific antibodies against certain food allergens in blood serum have been made to all patients. The results are presented in Table 7 below:

**Table 7**

| Results for established allergy to food allergens in 23 patients, presented as a percentage | | |
|---|---|---|
| **Allergen** | **Number of patients** | **%** |
| Cow's milk protein | 15 | 65,2 |
| Egg whites | 17 | 73,9 |
| Egg yolk | 6 | 26,1 |
| Soy | 1 | 4,3 |
| Pork | 1 | 4,3 |
| Veal | 2 | 8,7 |
| Chicken meat | 1 | 4,3 |
| Fish | 9 | 39,1 |
| Shrimps, mussels | 18 | 78,3 |
| Strawberry | 15 | 65,2 |
| Nuts - peanuts, almonds, hazelnuts | 19 | 82,6 |
| Cocoa | 14 | 60,9 |
| Goat milk protein | 1 | 4,3 |

Hypersensitivity to more than one food allergen in all patients has been established.

It is noteworthy the high rate of food allergy to peanuts, seafood, strawberry, egg whites, cocoa and cow's milk. Allergy to goat's milk has been established only in 1 patient. After establishing the type of food allergy, we recommended to all patients to stop the consumption of foods, containing the relevant food allergens. All patients, except the one with allergy to goat milk protein, have taken product P6 at a dose of 2 to 6 capsules daily for a period of 2 months depending on the severity of allergy. Patients have not taken anti-allergy medicines. Clinical symptoms of food allergy have subsided for a period of 2 to 6 weeks depending on the severity of the allergy. Our observations of the patients showed stopping the hair loss and emergence of new hair, normalization of the blood pressure without changing the antihypertensive therapy, reduction of bleeding and inflammation of the gums and normalization of prolactin levels, as patients have taken only product P6, they have followed the diet and have not taken medicines for hair loss, periodontal disease and regulation of the prolactin level.

The observation shows the effect of the combination of lactic acid product, containing strains of *Lactobacillus delbrueckii subsp. bulgaricus DMR1, Lactobacillus delbrueckii subsp. bulgaricus DMR2, Streptococcus thermophilus DMR3* and *Streptococcus hermophilus DMR4,* obtained through the method of the invention from goat's milk and Bulgarian rose oil on the control of the symptoms of food allergies

**Example 9:** Effect of a probiotic formula with strains of *Lactobacillus delbrueckii* subsp. *bulgaricus DMR1, Lactobacillus delbrueckii subsp. bulgaricus DMR2, Streptococcus thermophilus DMR3* and *Streptococcus thermophilus DMR4,* obtained through the method of the invention from dry skimmed cow's milk on the vaginal flora of women with vaginal discharge.

For the purpose of the study, a product, obtained through the method of the invention, from dry skimmed cow's milk is put together with dextrose monohydrate in rapidly dissolving vegetable capsules for a topical vaginal application, as the content of 1 capsule is: 250 mg lactic acid product and 70 mg dextrose monohydrate, as this product in that form was conditionally designated P3.

The study has been carried out in MC Evrozdrave and MC Gynart MMA Sofia for a period of 23 months. The clinical study included 32 women aged between 18 and 46 years with regular menstrual cycle who were not pregnant. All women, included in the study, were with vaginal discharge with different etiological genesis, as the diagnosis has been historically, clinically and paraclinically (microbiologically) based. All women were treated with specific medicines depending on the etiological genesis (Fluconazole, Tinidazole, Clindamycin, etc.) for a period of treatment from 5 to 10 days. Women were randomly divided into 3 groups. The first group comprising 10 women, has taken only etiological medication without a topical application of product P3. Product P3 was applied as a medical device in a dose of 1 capsule twice a day topically in the vagina for 5 days to 11 patients from the second group after completion of the anti-infective treatment. The application of product P3 to 11 women from the third group started along with the anti-infective treatment and continued 5 days after its end in a daily dose of 1 capsule 2 times applied topically in the vagina.

Gram-stained microscopic preparations were made to all patients aiming to count the number of lactobacilli in the vagina before the start of the etiological treatment and at the end of the treatment. Additional Gram-stained microscopic preparations were made to the patients from the second and the third group at the end of the treatment with product P3. The quantity of lactobacilli in the vagina of women was conditionally reported from (-) to (++++). The obtained results are summarized and shown in Table 8 below.

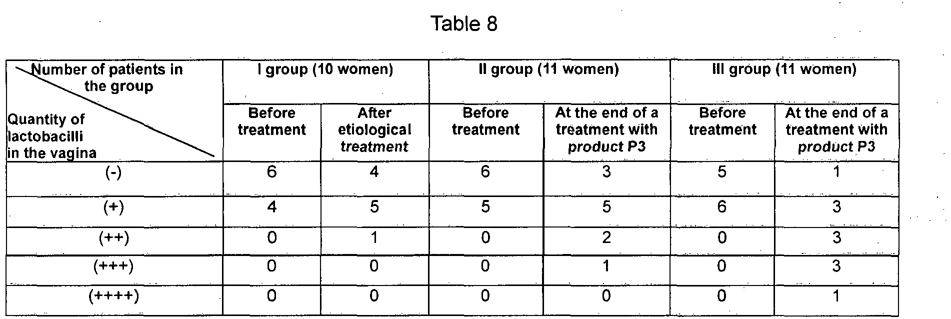

The results show an improvement of the flora of lactobacilli in the second group and a significant increase in the normal flora of lactobacilli in the third group. Improvement of the quantity of the lactobacilli is observed in the cases when product P3 is applied after the completion of the anti-infective treatment. The quantity of the lactobacilli in the normal flora is significantly the greatest when product P3 is applied during and up to 5 days after the anti-infective treatment of vaginal discharge.

The clinical study shows the efficiency of lactic acid product P3, obtained through the method of the Invention, containing strains *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4, applied topically, on restoration of the normal vaginal flora of lactobacilli of women, treated for vaginal discharge. The product P3 is most efficient when it is applied during and 5 days after the etiological treatment of vaginal discharge.

The study also shows that the strains *Lactobacillus delbrueckii* subsp. *bulgaricus* DMR1, *Lactobacillus delbrueckii* subsp. *bulgaricus* DMR2, *Streptococcus thermophilus* DMR3 and *Streptococcus thermophilus* DMR4 in the probiotic formula, prepared in accordance with the invention, colonize well woman's vagina when applied topically in woman's vagina.

## Claims

1. Process of preparation of a probiotic formula from cow's or goat's milk by fermentation and lyophilization, **characterized in that** a standardized and pasteurized cow's milk with 3.6% fat or sterilized pasteurized goat's milk with 3.2% fat is cultured by a probiotic cooperation of microorganism strains, including strain *Lactobacillus delbrueckii subsp. bulgsricus* DMR1, registered in CCM under Nº 8591/17.02.2015, strain *Lactobacillus delbrueckii subsp. bulgaricus* DMR2, registered in CCM under Nº 8592/ 17.02.2015, strain *Streptococcus thermophilus* DMR3, registered in CCM under Nº 8591/ 17.02.2015 and strain *Streptococcus thermophilus* DMR4, registered in CCM under Nº 8592/ 17.02.2015 in a ratio 1:0.005 in order to obtain a biomass which is added to standardized and pasteurized milk in a ratio 1: 0.001 and is left to ferment for 18 hours at a temperature of 43°C- 45°C, the resultant liquid starter culture is added to 17% water solution of dry cow's or fresh goat's milk in a ratio 3:1 together with a cryoprotectant bee honey and the obtained mixture is homogenized at a temperature of 41°C - 44°C, then it is fermented for 3 hours and then is cooled down to 24°C- 25°C, then is lyophilized and is stored at a temperature of 24°C.

2. Process of preparation of a probiotic formula according to Claim 1, **characterized in that** the strains Lactobacillus *delbrueckii subsp bulgaricus* DMR1, registered in CCM under Nº 8591/ 17.02.2015, Lactobacillus *delbrueckii subsp. bulgaricus* DMR2, registered in CCM under Nº 8592/ 17.02.2015, Streptococcus thermophilus DMR3, registered in CCM under Nº 8591/ 17.02.2015 and Streptococcus thermophilus DMR4, registered in CCM under Nº 8592/ 17.02.2015 are included in the probiotic cooperation in a ratio 1:1:0.5:0.5.

3. Process of preparation of a probiotic formula according to Claim 1, **characterized in that** the cryoprotectant bee's honey is previously heat treated.

4. Process of preparation of a probiotic formula according to Claims 1-3, **characterized in that** the cryoprotectant is put in the 17% water solution of dry cow's or goat's milk to which the liquid starter culture is added in a ratio 1:50.

5. Process of preparation of a probiotic formula according Claims 1-4, **characterized in that** the liquid starter culture is obtained also by rehydration of the lyophilized product from Claim 1 by water in a ratio water: dry product 1:0.5.

6. Probiotic formula, prepared according to Claims 1-5, **characterized in that** after storage for 24 months at a room temperature 24°C, it contains live cells from the probiotic cooperation in quantity from 1,1 x 10⁹cfu/g to 1,6 x 10⁹cfu/g.

7. Probiotic formula according to Claim 6, **characterized in that** after storage for 24 months at a room temperature 24°C, it contains live cells of strain *Streptococcus thermophilus DMR3,* registered in CCM under Nº8591 and strain *Streptococcus thermophilus DMR4,* registered in CCM under Nº 8592 in quantity from 4,1 x 10⁸ cfu/g to 1,1 x 10⁹ cfu/g, as well as live cells of strain *Lactobacillus delbrueckii ssp. Bulgaricus DMR1,* registered in CCM under Nº 8591 and strain *Lactobacillus delbrueckii* ssp. *Bulgaricus DMR2,* registered in CCM under Nº 8592 in quantity from 5,1 x 10⁸cfu/g to 8,4 x 10⁸cfu/g.

8. Probiotic formula according to claims 6 and 7 in combination with silymarin, dextrose monohydrate and magnesium stearate in a ratio 15:10:1:17 for use in the restoration of liver structure in patients with liver cirrhosis in addition to the basic treatment for the restoration of the liver structure in patients with liver cirrhosis.

9. Probiotic formula according to claims 6 and 7 in combination with silymarin, dextrose monohydrate and magnesium stearate in a ratio 15:10:1:17 for use in the restoration of the functions of the liver cells in patients with alcoholic disease in addition to the basic treatment for restoration of the functions of the liver cells in patients with alcoholic disease.

10. Probiotic formula from goat's milk according to claims 6 and 7 containing Bulgarian rose oil, dextrose monohydrate and magnesium stearate in a ratio 25:1:2:3 for use in controlling the symptoms of food allergy.

11. Probiotic formula according to claims 6 and 7 for use in recovering the normal vaginal lactobacillus microflora of woman.

12. Probiotic formula for use according to claim 11 wherein the probiotic formula is a medical device.

## Patentansprüche

1. Verfahren zur Herstellung von eine probiotischen Formel aus Kuhmilch oder Ziegenmilch mittels Gärung und Lyophilisation, **dadurch gekennzeichnet, dass** eine standardisierte und pasteurisierte Kuhmilch mit einem Fettgehalt von 3.6% oder eine sterilisierte pasteurisierte Ziegenmilch mit einem Fettgehalt von 3.2% mittels einer probiotischen Kooperation von Stämmen von Mikroorganismen kultiviert werden, beinhalten den Stamm *Lactobacillus delbrueckii subsp. bulgaricus DMR1,* registriert im CCM unter Nr. 8591/17.02.2015, Stamm *Lactobacillus delbrueckii subsp. bulgaricus DMR2,* registriert im CCM unter Nr. 8592/17.02.2015, Stamm *Streptococcus thermophilus DMR3,* registriert im CCM unter Nr. 8591/17.02.2015 und Stamm *Streptococcus thermophilus DMR4,* registriert im CCM unter Nr. 8592/17.02.2015 in einem Verhältnis 1: 0.005, um eine Biomasse herzustellen, die der standardisierten und pasteurisierten Milch in einem Verhältnis 1:0.001 hinzugefügt und binnen 18 Stunden bei einer Temperatur von 43°C - 45°C gegoren wird, die so resultierende flüssige Starterkultur wird zu einer 71%-igen Wasserlösung von trockener Kuhmilch oder frischer Ziegenmilch in einem von Verhältnis 3: 1 gegeben, zusammen mit einem kryoprotektiven Bienenhonig und die erhaltene Mischung wird bei einer Temperatur von 41°C - 44°C homogenisiert, wonach sie im Laufe von 3 Stunden gegoren und danach bis zu 24°C - 25°C abgekühlt wird, und nachher lyophilisiert und bei einer Temperatur von 24°C aufbewahrt wird.

2. Verfahren zur Herstellung von eine probiotischen Formel gemäß Anspruch 1, sich **dadurch gekennzeichnet, dass** die Stämme *Lactobacillus delbrueckii subsp. bulgaricus DMR1,* registriert im CCM unter Nr. 8591/17.02.2015, *Lactobacillus delbrueckii subsp. bulgaricus DMR2,* registriert im CCM unter Nr. 8592/17.02.2015, *Streptococcus thermophilus DMR3,* registriert im CCM unter Nr. 8591/17.02.2015 und *Streptococcus thermophilus DMR4,* registriert im CCM unter Nr. 8592/17.02.2015 in der probiotischen Kooperation in einem Verhältnis 1:1:0.5:0.5 eingeschlossen sind.

3. Verfahren zur Herstellung von eine probiotischen Formel gemäß Anspruch 1, sich **dadurch gekennzeichnet, dass** der Kryoprotektor Bienenhonig im Voraus thermisch bearbeitet wurde.

4. Verfahren zur Herstellung von eine probiotischen Formel gemäß Ansprüchen 1-3, sich **dadurch gekennzeichnet, dass** der Kryoprotektor in eine 17%-ige Wasserlösung von trockener Kuhmilch oder Ziegenmilch gestellt wird, wo auch die flüssige Starterkultur im Verhältnis 1: 50 hinzugefügt wurde.

5. Verfahren zur Herstellung von eine probiotischen Formel gemäß Ansprüchen 1-4, sich **dadurch gekennzeichnet, dass** die flüssige Starterkultur auch mittels Rehydrierung des lyophilisierten Produkts aus Anspruch 1 mit Wasser in einem Verhältnis Wasser: Trockenprodukt 1: 0.5 erzeugt wird.

6. Probiotische Formel, hergestellt gemäß den Ansprüchen 1-5, sich **dadurch gekennzeichnet, dass** nach einer Aufbewahrung im Laufe von 24 Monaten bei Zimmertemperatur 24°C, diese lebende Zellen von der probiotischen Kooperation in einer Menge von 1.1 x 10⁹ cfu/g bis 1.6 x 10⁹ cfu/g enthält.

7. Probiotische Formel nach Anspruch 6, sich **dadurch gekennzeichnet, dass** nach einer Aufbewahrung im Laufe von 24 Monaten bei Zimmertemperatur 24°C, diese lebendige Zellen des Stamms *Streptococcus thermophilus DMR3,* registriert im RZVM unter Nr. 8591 und des Stamms *Streptococcus thermophilus DMR4,* registriert im RZVM unter Nr. 8592 in einer Menge von 4.1 x 10⁸ cfu/g bis 1.1 x 10⁹ cfu/g sowie lebendige Zellen des Stamms *Lactobacillus delbrueckii ssp. bulgaricus DMR1,* registriert im RZVM unter Nr. 8591 und des Stamms *Lactobacillus delbrueckii subsp. bulgaricus DMR2,* registriert im RZVM unter Nr. 8592 in einer Menge von 5.1 x 10⁸ cfu/g bis 8.4 x 10⁸ cfu/g, enthält.

8. Probiotische Formel gemäß Ansprüchen 6 und 7 in einer Kombination mit Silymarin, Dextrose Monohydrat und Magnesiumstearat im Verhältnis 15:10:1:17 zur Anwendung (use- in English) bei der Regeneration der Leberstruktur bei Patienten mit Leberzirrhose als Ergänzung der Grundbehandlung zur Regeneration der Leber Struktur bei Patienten mit Leberzirrhose.

9. Probiotische Formel gemäß Ansprüchen 6 und 7 in einer Kombination mit Silymarin, Dextrose Monohydrat und Magnesiumstearat im Verhältnis 15:10:1:17 zur Anwendung bei der Wiederherstellung der Funktionen der Leberzellen bei Patienten mit Alkoholkrankheit als Ergänzung der Grundbehandlung zur Wiederherstellung der Funktionen der Leberzellen bei Patienten mit Alkoholkrankheit.

10. Probiotische Formel aus Ziegenmilch gemäß Ansprüchen 6 und 7, enthaltend bulgarisches Rosenöl, Dextrose Monohydrat und Magnesiumstearat im Verhältnis 25:1:2:3 zur Anwendung bei der Kontrolle der Symptome einer Nahrungsmittelallergie.

11. Probiotische Formel gemäß Ansprüchen 6 und 7 zur Anwendung bei der Wiederherstellung der normalen vaginalen lactobacillaren Mikroflora von Frauen.

12. Probiotische Formel zur Anwendung gemäß Anspruch 11, wo die probiotische Formel ein medizinisches Erzeugnis ist.

## Revendications

1. Procédé de préparation d'une formule probiotique à partir de lait de vache ou de chèvre par fermentation et lyophilisation, **caractérisé en ce qu'**un lait de vache standardisé et pasteurisé à 3,6 % de matière grasse ou un lait de chèvre pasteurisé stérilisé à 3,2 % de matière grasse est cultivé par une coopération probiotique de souches de micro-organismes, incluant la souche *Lactobacillus delbrueckii subsp. bulgaricus DMR1,* enregistrée dans la CCM sous le n° 8591/17.02.2015, la souche *Lactobacillus delbrueckii subsp. bulgaricus DMR2,* enregistrée dans la CCM sous le n° 8592/17.02.2015, la souche *Streptococcus thermophilus DMR3,* enregistrée dans la CCM sous le n° 8591/17.02.2015 et la souche *Streptococcus thermophilus DMR4,* enregistrée au CCM sous le numéro 8592/17.02.2015 dans un rapport de 1:0.005 afin d'obtenir une biomasse qui est ajoutée au lait standardisé et pasteurisé dans un rapport de 1:0,001 et est laissée à fermenter pendant 18 heures à une température de 43°C- 45°C, la culture liquide de départ qui en résulte est ajoutée à une solution aqueuse à 17% de lait de vache ou de chèvre frais dans un rapport 3:1 avec un miel d'abeille cryoprotecteur et le mélange obtenu est homogénéisé à une température de 41°C - 44°C, puis il est fermenté pendant 3 heures et ensuite refroidi à 24°C- 25°C, puis il est lyophilisé et stocké à une température de 24°C.

2. Procédé de préparation d'une formule probiotique selon la revendication 1, **caractérisé en ce que** les souches *Lactobacillus delbrueckii subsp. bulgaricus DMR1,* enregistrée dans la CCM sous le nº 8591/17.02.2015, *Lactobacillus delbrueckii subsp. bulgaricus DMR2,* enregistrée dans la CCM sous le nº 8592/17.02.2015, *Streptococcus thermophilus DMR3,* enregistrée dans la CCM sous le nº 8591/17.02.2015 et *Streptococcus thermophilus DMR4,* enregistrée dans la CCM sous le numéro 8592/17.02.2015 sont incluses dans la coopération probiotique dans un rapport 1:1:0,5:0,5.

3. Procédé de préparation d'une formule probiotique selon la revendication 1, **caractérisé en ce que** le miel d'abeille cryoprotecteur est préalablement traité thermiquement.

4. Procédé de préparation d'une formule probiotique selon les revendications 1 à 3, **caractérisé en ce que** le cryoprotecteur est mis dans une solution aqueuse à 17% de lait sec de vache ou de chèvre à laquelle la culture liquide de départ est ajoutée dans un rapport 1:50.

5. Procédé de préparation d'une formule probiotique selon les revendications 1 à 4, **caractérisé en ce que** la culture de départ liquide est obtenue également par réhydratation du produit lyophilisé selon la revendication 1 par de l'eau dans un rapport eau/produit sec de 1:0,5.

6. Formule probiotique, préparée selon les revendications 1 à 5, **caractérisée en ce qu'**elle contient, après un stockage de 24 mois à une température ambiante de 24°C, des cellules vivantes issues de la coopération probiotique comprise entre 1,1x10⁹ ufc/g et 1,6x10⁹ ufc/g.

7. Formule probiotique selon la revendication 6, **caractérisée en ce qu'**elle contient, après un stockage de 24 mois à une température ambiante de 24°C, des cellules vivantes de la souche *Streptococcus thermophilus DMR3,* enregistrée dans la CCM sous le nº 8591 et de la souche *Streptococcus thermophilus DMR4,* enregistrée dans la CCM sous le nº 8592 comprise entre 4,1x10⁸ ufc/g et 1,1x 10⁹ ufc/g, ainsi que des cellules vivantes de la souche *Lactobacillus delbrueckii ssp. Bulgaricus DMR1,* enregistrée dans la CCM sous le nº 8591 et la souche *Lactobacillus delbrueckii ssp. Bulgaricus DMR2,* enregistrée dans la CCM sous le nº 8592, en quantité comprise entre 5,1x10⁸ ufc/g et 8,4 x 10⁸ ufc/g.

8. Formule probiotique selon les revendications 6 et 7 en combinaison avec de la silymarine, du monohydrate de dextrose et du stéarate de magnésium dans un rapport de 15:10:1:17 utilisée pour la restauration de la structure du foie chez les patients atteints de cirrhose du foie en plus du traitement de base pour la restauration de la structure du foie chez les patients atteints de cirrhose du foie.

9. Formule probiotique selon les revendications 6 et 7 en combinaison avec de la silymarine, du monohydrate de dextrose et du stéarate de magnésium dans un rapport de 15:10:1:17 utilisée pour la restauration des fonctions des cellules hépatiques chez les patients atteints de maladie alcoolique en plus du traitement de base pour la restauration des fonctions des cellules hépatiques chez les patients atteints de maladie alcoolique.

10. Formule probiotique à partir de lait de chèvre selon les revendications 6 et 7, incluant de l'huile essentielle de rose bulgare, du monohydrate de dextrose et du stéarate de magnésium dans un rapport de 25:1:2:3 utilisée pour contrôler les symptômes d'une allergie alimentaire.

11. Formule probiotique pour son utilisation selon les revendications 6 et 7 pour la restauration de la flore vaginale normale de lactobacilles chez la femme.

12. Formule probiotique pour son utilisation selon la revendication 11, dans laquelle la formule probiotique est un dispositif médical.
